Europäisches Patentamt

**European Patent Office**

Office européen des brevets

(11) Veröffentlichungsnummer: **0 120 494**
**B1**

(12) **EUROPÄISCHE PATENTSCHRIFT**

(45) Veröffentlichungstag der Patentschrift:
**28.10.87**

(21) Anmeldenummer: **84103324.4**

(22) Anmeldetag: **26.03.84**

(51) Int. Cl.⁴: **C 07 D 417/12**

(54) **Feinkristallines Isoxicam.**

(30) Priorität: **26.03.83 DE 3311165**

(43) Veröffentlichungstag der Anmeldung:
**03.10.84 Patentblatt 84/40**

(45) Bekanntmachung des Hinweises auf die Patenterteilung:
**28.10.87 Patentblatt 87/44**

(84) Benannte Vertragsstaaten:
**AT BE CH DE FR GB IT LI LU NL SE**

(56) Entgegenhaltungen:
**EP - A - 0 091 044**

**JOURNAL OF MEDICINAL CHEMISTRY, Band 25, Nr. 1, Januar 1982, Seiten 12-18; H. ZINNES et al.: "Isoxicam and related 4-hydroxy-N-isoxazolyl-2H-1,2-benzothiazine-3-carboxamide 1,1-dioxides. Potent nonsteroidal antiinflammatory agents"**

**Die Akte enthält technische Angaben, die nach dem Eingang der Anmeldung eingereicht wurden und die nicht in dieser Patentschrift enthalten sind.**

(73) Patentinhaber: **GÖDECKE AKTIENGESELLSCHAFT, Salzufer 16, D-1000 Berlin 10 (DE)**

(72) Erfinder: **Herrmann, Wolfgang, Dr., Zum Baumgarten 13, D-7802 Merzhausen (DE)**
Erfinder: **Gebhardt, Uwe, Dr., Von Bayer Strasse 19, D-7808 Waldkirch (DE)**

## Beschreibung

Isoxicam ist ein aus der DE-CS 2 208 351 bekannter Wirkstoff aus der Gruppe der 1,2-Benzothiazin-1,1-dioxide. Er wird als Antirheumatikum verwendet.

Da Isoxicam relativ schwer wasserlöslich ist, bestehen bei oraler Gabe Schwierigkeiten bezüglich der Bioverfügbarkeit und es muss durch geeignete Massnahmen dafür gesorgt werden, dass eine ausreichende und vor allem von äusseren Bedingungen und vom Patienten unabhängige und gleichmässige Resorption stattfindet.

In der Regel wird bei schwer resorbierbaren Stoffen versucht, die beim Herstellungs-, bzw. Reinigungsprozess des Wirkstoffs anfallenden relativ grobkörnigen Kristalle entweder mechanisch auf eine brauchbare Korngrösse zu zerkleinern oder man wählt die Kristallisationsbedingungen so, dass von vornherein Kristalle der gewünschten Grösse anfallen.

Bei der mechanischen Zerkleinerung, beispielsweise bei der Mikronisierung mittels Luftstrahlmühlen (jet-mills), ist es praktisch nicht möglich, eine einheitliche Korngrösse zu erzielen, da der Mahlvorgang Bruchstücke der ursprünglichen Kristalle in den verschiedensten Grössen erzeugt. Darüberhinaus sind Luftstrahlmühlen sowohl von der Anschaffung her als auch im Betrieb kostenaufwendig und erzielen nur einen relativ geringen Substanzdurchsatz in der Zeiteinheit.

Die Umkristallisation schwer löslicher Stoffe führt auch dann, wenn an sich brauchbare Bedingungen bezüglich Lösungsmittel und Kristallisationsbedingungen ermittelt werden können, zu hohen Substanzverlusten, weil eine vollständige Rückgewinnung aus der Mutterlauge nur unter hohen Kosten möglich ist. Es ist hierbei auch schwierig, einheitliche Kristallgrössen zu erzielen.

Bei Umfällungsverfahren, die sehr fein verteilte Partikel ergeben können, fällt der Niederschlag in den allermeisten Fällen amorph oder so feinkristallin an, dass die weitere Handhabung (Zentrifugieren, Filtration, Trocknung, Weiterverarbeitung) extrem erschwert wird. In der nicht vor publizierten EP-A-91044 ist ein Eintopfverfahren zur Herstellung von Isoxicam beschrieben, bei dem der Wirkstoff aus dem Reaktionsgemisch unmittelbar in einer Reinheit von 96% ausgefällt wird und aus Aceton umkristallisiert werden muss.

Es wurde nun überraschend gefunden, dass Isoxicam kristallin und in gut verarbeitbarer und hervorragend resorbierbarer Form anfällt, wenn man den Wirkstoff in einem Gemisch, bestehend aus einem mit Wasser mischbaren Lösungsmittel und einer wässrigen Base löst, wobei soviel Base zugegeben wird, dass sich nach Lösung des Wirkstoffs ein pH-Wert von 8-9 einstellt, die so erhaltene Lösung nach Filtration durch Zugabe von verdünnter Säure unter starkem Rühren auf einen pH-Wert von 4-6 bringt, den ausgefallenen Niederschlag von der Mutterlauge abtrennt, mit Wasser wäscht und bei 50-60°C bis zur Gewichtskonstanz trocknet.

Als Lösungsmittel eignen sich z.B. niedere Alkohole, Tetrahydrofuran, Dimethylformamid, Dioxan und insbesondere Aceton.

Als Base hat sich besonders Ammoniaklösung bewährt. Es ist jedoch auch möglich, verdünnte, vorzugsweise 1-normale wässrige Kali- oder Natronlauge zu verwenden.

Wird rohes Isoxicam als Ausgangsmaterial zur Herstellung des erfindungsgemässen Produktes verwendet, so sollte die vor der Fällung notwendige Filtration am besten nach vorheriger Behandlung mit Aktivkohle erfolgen, da dadurch ein besonders reines Produkt erzielt wird.

Als Fällungsmittel eignen sich sowohl organische Säuren wie Essigsäure oder Propionsäure als auch Mineralsäuren wie Salz- oder Schwefelsäure in etwa 1-normaler Verdünnung.

Es ist als sehr überraschend zu bezeichnen, dass bei erfindungsgemässer Durchführung der Fällung Isoxicam in einer feinkristallinen Form anfällt, die sich obendrein durch eine sehr gleichmässige Korngrössenverteilung auszeichnet.

Die Substanz liegt als weisses, mehliges Pulver in einer Korngrösse zwischen 1 und 30 μm (2,0 μm < 90% < 25 μm) vor und entspricht damit etwa dem wesentlich aufwendiger hergestellten Isoxicam, das mittels Strahlmühle mikronisiert wurde (1 μm < 90% < 15 μm). Die Korngrössenanalysen wurden mit dem Coulter Counter durchgeführt.

Es ist überraschend, dass das erfindungsgemässe Produkt trotz der feinkörnigen Struktur nicht zum Verkleben oder Verbacken neigt.

Versuche haben gezeigt, dass ein wesentlicher Grund hierfür darin besteht, dass ausschliesslich Wasser für die Nachwäsche, z.B. auf der Zentrifuge, verwendet wird. Bei Verwendung organischer Lösungsmittel erhält man, auch unter Verdünnung mit Wasser, harte Agglomerate, die kaum zerkleinert werden können.

Wenn jedoch nach dem Abschleudern der Mutterlauge nur mit Wasser gewaschen wird, erhält man ein lockeres Pulver, das sich schon beim Sieben völlig verteilt.

Unerwarteterweise sind unabhängig von der Korngrösse die Lösungseigenschaften des erfindungsgemäss hergestellten, feinkristallinen Wirkstoffes wesentlich besser als die des mikronisierten Wirkstoffes.

Das erfindungsgemässe feinkristalline Isoxicam löst sich in einer Pufferlösung von pH 9 bei 37°C doppelt so schnell wie Isoxicam normaler Korngrösse (10 – 200 μm; nicht mikronisiert) und in einer um 25% kürzeren Zeit als mikronisiertes Isoxicam.

Ein ähnlicher Effekt zeigt sich bei den mit verschiedenen Isoxicam-Qualitäten hergestellten Tabletten und Gelatine-Kapseln: Die Darreichungsformen mit mikronisiertem Wirkstoff ergeben experimentell (Sartorius Dissolution Model) eine um 50% langsamere Auflösung als die mit erfindungsgemäss hergestellten feinkristallinen Wirkstoff hergestellten Formen. Da die mitt-

lere Korngrösse des erfindungsgemäss herge-stellten mikrokristallinen Isoxicams etwas höher liegt als die des mikronisierten Wirkstoffs, wäre an sich eine schnellere Auflösung des mikronisierten Isoxicams zu erwarten gewesen.

Studien zur Bioverfügbarkeit zeigten ebenfalls eine signifikante Überlegenheit des erfindungsgemässen, feinkristallinen Isoxicams. Tabletten und Kapseln mit diesem Wirkstoff ergaben bei gleicher galenischer Formulierung eine um 100 Prozent höhere relative Bioverfügbarkeit als Tabletten und Kapseln mit mikronisiertem Wirkstoff; bei Zäpfchen wurde eine um 23% bessere Bioverfügbarkeit gefunden, wenn das vorliegende feinkristalline Isoxicam anstelle von mikronisiertem Isoxicam zu ihrer Herstellung verwendet worden war.

Das folgende Beispiel dient der näheren Erläuterung der Erfindung:

Beispiel

1 kg rohes Isoxicam wird in einem Gemisch von 8 l Aceton und 8 l 0,5 n wässriger Ammoniaklösung unter Rühren bei Raumtemperatur gelöst. Es stellt sich ein pH-Wert von ca. 8 ein. Anschliessend wird mit 0,3 kg Aktivkohle 15 Minuten gerührt und die Lösung dann filtriert. In das Filtrat lässt man unter gutem Rühren 4,8 l 1 n Essigsäure einlaufen, wobei ein pH von 6 – 6 resultiert, rührt kurz nach und trennt den Niederschlag durch Zentrifugieren von der Mutterlauge ab. Das Kristallisat wird dreimal mit je 2 l entmineralisiertem Wasser gewaschen und anschliessend im Umlufttrockenschrank bei 55 - 60°C bis zur Gewichtskonstanz getrocknet.

Die trockene Substanz wird durch ein Sieb mit 0,5 mm Maschenweite (35 mesh) gegeben, wobei eventuell vorhandene lockere Klumpen von selbst zerfallen.

Ausbeute: 860 g = 86% d.Th.
　　　　　Reinheit nach DC und HPLC > 99%
　　　　　Korngrösse zwischen 1 und 30μm.

Die Fällung kann auch unter Verwendung von Mineralsäuren durchgeführt werden und ebenso in umgekehrter Weise dergestalt, dass man die basische Lösung von Isoxicam in die vorgelegte wässrige Säure einlaufen lässt. Das Mischen der beiden Lösungen kann auch in einem kontinuierlichen Verfahren erfolgen. Als Ausgangsprodukt kann auch reines Isoxicam verwendet werden. In diesem Falle ist eine Behandlung der Lösung mit Kohle nicht notwendig, und die Ausbeute liegt bei etwa 95%.

**Patentansprüche**

1. Feinkristallines Isoxicam mit einer Korn-grösse von 1 – 30 μm, wobei 90% in dem Bereich zwischen 2 und 25 μm liegen, dadurch herge-stellt, dass man den Wirkstoff in einem Gemisch bestehend aus einem mit Wasser mischbaren Lösungsmittel und einer wässrigen Base löst, wobei so viel Base zugegeben wird, dass sich nach Lösung des Wirkstoffs ein pH-Wert von 8 – 9 einstellt, die so erhaltene Lösung durch Zugabe von verdünnter Säure unter gutem Rühren auf einen pH-Wert von 4 – 6 bringt, den ausgefallenen Niederschlag von der Mutterlauge abtrennt, mit Wasser wäscht und bei 50 - 60°C trocknet.

2. Umgefälltes Isoxicam nach Anspruch 1, dadurch gekennzeichnet, dass als Lösungsmittel Aceton und als Base wässrige Ammoniaklösung verwendet worden ist.

3. Umgefälltes Isoxicam nach Anspruch 1 und 2, dadurch gekennzeichnet, dass die Lösung vor Zugabe der Säure filtriert worden ist.

4. Umgefälltes Isoxicam nach Anspruch 1 bis 3 dadurch gekennzeichnet, dass als verdünnte Säure die Essigsäure verwendet worden ist.

5. Verfahren zur Herstellung von feinkristallinem Isoxicam, mit einer Korngrösse von 1 – 30 μm, wobei 90% in dem Bereich zwischen 2 und 25 μm liegen, dadurch gekennzeichnet, dass man den Wirkstoff in einem Gemisch bestehend aus einem mit Wasser mischbaren Lösungsmittel und einer wässrigen Base löst, wobei so viel Base zugegeben wird, dass sich nach Lösung des Wirkstoffs etwa ein pH-Wert von 8 – 9 einstellt, die so erhaltene Lösung durch Zugabe von verdünnter Säure unter gutem Rühren auf einen pH-Wert von 4 – 6 bringt, den ausgefallenen Niederschlag von der Mutterlauge abtrennt, mit Wasser wäscht und bei 50 - 60°C trocknet.

6. Verfahren nach Anspruch 5, dadurch gekennzeichnet, dass als Lösungsmittel Aceton und als Base wässrige Ammoniaklösung verwendet wird.

7. Verfahren nach Anspruch 5 und 6, dadurch gekennzeichnet, dass die Lösung vor Zugabe der Säure filtriert wird.

8. Verfahren nach Anspruch 5 bis 7, dadurch gekennzeichnet, dass als verdünnte Säure die Essigsäure verwendet wird.

**Claims**

1. Fine crystalline isoxicam with a grain size of 1 – 30 μm., whereby 90% lie in the range between 2 and 35 μm., prepared in that one dissolves the active material in a mixture consisting of a solvent miscible with water and an aqueous base, whereby so much base is added that, after dissolving of the active material, a pH value of 8 – 9 is obtained, brings the solution so obtained to a pH value of 4 – 6 by the addition of dilute acid, with good stirring, separates the precipitate obtained from the mother liquor, washes with water and dries at 50 – 60°C.

2. Reprecipitated isoxicam according to claim 1, characterised in that acetone is used as solvent and aqueous ammonia solution as base.

3. Reprecipitated isoxicam according to claims 1 and 2, characterised in that the solution is filtered before addition of the acid.

4. Reprecipitated isoxicam according to claims 1 to 3, characterised in that acetic acid is used as dilute acid.

5. Process for the preparation of fine crystalline isoxicam with a grain size of 1 – 30 μm., whereby 90% lie in the range between 2 and 25 μm., characterised in that one dissolves the active material in a mixture consisting of a solvent miscible with water and an aqueous base, whereby so much base is added that, after dissolving of the active material, a pH value of 8 – 9 is obtained, brings the solution so obtained to a pH value of 4 – 6 by the addition of dilute acid, with good stirring, separates off the precipitate obtained from the mother liquor, washes with water and dries at 50 – 60°C.

6. Process according to claim 6, characterised in that acetone is used as solvent and aqueous ammonia solution as base.

7. Process according to claims 5 and 6, characterised in that the solution is filtered before addition of the acid.

8. Process according to claims 5 to 7, characterised in that acetic acid is used as dilute acid.

**Revendications**

1. Isoxicame finement cristallisé présentant une dimension granulaire de 1 – 30 μm, ladite dimension étant pour 90% de cet isoxicame comprise entre 2 et 25 μm, caractérisé en ce qu'on dissout le produit actif dans un mélange d'un solvant miscible à l'eau et d'une base aqueuse, employée dans une quantité qui confère à la solution, après dissolution du produit actif, un pH de 8 – 9; on ajuste la solution ainsi obtenue à un pH de 4 – 6 par l'adjonction d'acide dilué et sous bonne agitation, on sépare le précipité de la solution mère, on lave à l'eau et on sèche à 50 – 60°C.

2. Isoxicame reprécipité selon la revendication 1, caractérisé en ce que l'on a employé l'acétone comme solvant et une solution aqueuse d'ammoniac en tant que base.

3. Isoxicame reprécipité selon la revendication 1 et 2, caractérisé en ce que la solution a été filtrée préalablement à l'addition de l'acide.

4. Isoxicame reprécipité selon la revendication 1 à 3, caractérisé en ce que l'on a employé l'acide acétique comme acide dilué.

5. Procédé pour préparer de l'isoxicame finement cristallisé, présentant une dimension granulaire de 1 à 30 μm, ladite dimension étant pour 90% de cet isoxicame comprise entre 2 et 25 μm, caractérisé en ce qu'on dissout le produit actif dans un mélange d'un solvant miscible à l'eau et d'une base aqueuse, employée dans une quantité qui confère à la solution, après dissolution du produit actif, un pH de 8 – 9; on ajuste la solution ainsi obtenue à un pH de 4 – 6 par l'adjonction d'acide dilué et sous bonne agitation, on sépare le précipité de la solution mère, on lave à l'eau et on sèche à 50 – 60°C.

6. Procédé selon la revendication 5, caractérisé en ce que l'on emploie l'acétone comme solvant et une solution aqueuse d'ammoniac en tant que base.

7. Procédé selon la revendication 5 et 6, caractérisé en ce que la solution est filtrée avant l'addition de l'acide.

8. Procédé selon la revendication 5 à 7, caractérisé en ce que l'on emploie l'acide acétique comme acide dilué.